# EUROPEAN PATENT SPECIFICATION

(11) **EP 1 409 741 B1**
(45) Date of publication and mention of the grant of the patent: **18.01.2006**
(21) Application number: 02758903.5
(22) Date of filing: 25.07.2002
(51) Int. Cl.: C12Q 1/68, G01N 27/26

(54) **DETECTION METHOD OF NUCLEIC ACID HYBRIDIZATION**
NACHWEISVERFAHREN DER NUKLEINSÄUREHYBRIDISIERUNG
METHODE DE DETECTION D'HYBRIDATION D'ACIDES NUCLEIQUES

(30) Priority: 25.07.2001 KR 2001044971; 23.07.2002 KR 2002043389
(43) Date of publication of application: 21.04.2004
(73) Proprietor: Posco, Pohang-shi, Kyungsangbuk-do 790-300 (KR); Postech Foundation, Pohang-City, Kyungsangbuk-do 790-784 (KR)
(72) Inventor: HAHN, Jong-Hoon, 790-751 Pohang-city, Kyungsanbuk-do (KR); PARK, NoKyoung 19-202, Postech Found. Dormitory, 790-330 Pohang-city, Kyungsangbuk-do (KR)
(74) Representative: Ritthaler, Wolfgang
(86) International application number: PCT/KR2002/001402
(87) International publication number: WO 2003/010338

(56) References cited:
- WO-A1-01/11080
- WO-A1-99/29711
- US-A- 5 968 745
- DATABASE WPI Week 200031, Derwent Publications Ltd., London, GB; AN 2000-353333, XP002972910 & JP 2000 083647 A (TOSHIBA KK) 28 March 2000

## Description

### BACKGROUND OF THE INVENTION

### (a) Field of the Invention

The present invention relates to a detection method of nucleic acid hybridization, and more particularly, to an electrochemical detection method of change of charge state of nucleic acid using a nucleic acid binding material specific to single-stranded nucleic acid or a double-stranded nucleic acid.

### (b) Description of the Related Art

The detection of nucleic acid hybridization is the most important technique in the field of nucleic acid chips. The general detection method is a laser induced fluorescence spectroscopic method, which detects a light stimulated by irradiating a laser to a fluorescence-labeled nucleic acid. Affymetrix and Nanogen offer DNA chips that have been tested based on the laser induced fluorescence spectroscopic method. However several problems are raised: (a) it is cumbersome to label nucleic acid with fluorescent molecules; (b) the process is complex; and (c) florescent molecules are expensive.

Recently, detection methods of nucleic acid hybridization without fluorescence use have been studied, and methods using surface plasmon resonance, quartz crystal microbalance, mass spectrometry, and electrochemistry have been developed.

However, except for the electrochemical method, these methods still have problems such as high cost and laborious processes, while the method using electrochemistry is simple so that everybody can carry it out without professional knowledge and most of the equipment used can be found at a low price.

### SUMMARY OF THE INVENTION

It is an object of the present invention to provide an electrochemical method for detecting nucleic acid hybridization.

It is another object of the present invention to provide a detection method of nucleic acid hybridization that can be employed simply and with low cost.

In order to achieve these objects, this invention provides a detection method of nucleic acid hybridization, comprising:
(a) preparing an oligo-plate by fixing a capture probe onto a working electrode surface;
(b) hybridizing the capture probe with a target probe;
(c) reacting a nucleic acid binding material specific to single-stranded nucleic acid or a double-stranded nucleic acid and
(d) measuring the change of charge state on the working electrode surface with an electrochemical method under an electroactive electrolyte condition.

### BRIEF DESCRIPTION OF THE DRAWINGS

Figure 1 schematically shows an oligo-plate preparation scheme. ,
Figures 2 and 3 show a multi-array.
Figure 4a shows an oligo-plate with hybridized nucleic acid.
Figure 4b shows a neutralization effect of negative charges on an electrode surface by intercalation.
Figure 5a shows an oxidation/reduction reaction on an electrode surface where the negative charge is neutralized by intercalation.
Figure 5b shows electrostatic repulsion of a negatively charged electrode surface against [Fe(CN)₆]³⁻.
Figure 6a is a cyclic voltammogram of a working electrode before intercalation.
Figure 6b is a cyclic voltammogram of a working electrode fixed with a single strand DNA after intercalation.
Figure 6c is a cyclic voltammogram of a working electrode fixed with a DNA hybrid after intercalation.
Figure 7 is a cyclic voltammogram of a working electrode after intercalation by an acridine compound.

### DETAILED DESCRIPTION OF THE PRESENT INVENTION

The present invention is related to a detection method of nucleic acid hybridization comprising intercalating a single-stranded nucleic acid or a double-stranded nucleic acid fixed on a working electrode surface with a specific nucleic acid binding material, and measuring the negative charge change of single stranded nucleic acid or the double-stranded nucleic acid.

The nucleic acid of the present invention is DNA or RNA.

The term "hybrid" refers to a double-stranded nucleic acid having a complementary binding.

The nucleic acid binding material of the present invention is a chemical compound that can be covalently or non-covalently bonded to a double-stranded nucleic acid or a single-stranded nucleic acid. The nucleic acid binding material can be selected from the group consisting of a chemical capable of being inserted into the grooves of a DNA duplex, a chemical capable of intercalation between a stacked base pair, a single-stranded nucleic acid specific binding chemical, a double-stranded nucleic acid specific binding chemical, and a major or minor groove of a DNA-duplex-specific binding chemical. The nucleic acid binding materials are charged molecules, and thus their binding on nucleic acids changes the charge state of the working electrode surface and more preferably, the nucleic acid binding material is a chemical having a positive charge and specificity between a double-stranded nucleic acid and a single-stranded nucleic acid.

Examples of nucleic acid binding material are an intercalator, an intercalating fluorescent dye, an acridine compound, chloroquine, quinine, novanatrone, doxorubicin, a single strand binding protein, and Rec A protein.

The intercalator has a specificity to double stranded nucleic acid and include an intercalating fluorescent dye, an acridine compound, chloroquine, quinine, novanatrone and doxorubicin

The intercalating fluorescent dye can be selected from the group consisting of 1,1'-[1,3-propanediylbis[(dimethyliminio) -3,1-propanediyl]]bis[4-[(3-methyl-2(3H)-benzoxazolylidene) methyl]]-, tetraiodide(YOYO -1), 1,1'-[1,3-propanediylbis[(dimethyliminio) -3,1-propanediyl]]bis[4-[3-(3-methyl-2(3H)-benzoxazolylidene) -1-propenyl]]-,tetraiodide(YOYO -3), 4-[(3-methyl-2(3H)-benzoxazolylidene) methyl]-1-[3-(trimethylammonio) propyl]-, diiodide(YO-PRO -1), 4-[3-(3-methyl-2(3H) - benzoxazolylidene) -1-propenyl]-1-(3-(trimethylammonio) propyl]-, diiodide(YO-PRO -3), 2,2'-[1,3-propanediylbis [(dimethyliminio) -3,1-propanediyl-1 (4H)-pyridinyl-4-ylidenemethylidyne]]bis[3- methyl]-,tetraiodide(POPOTM-1), 2,2'-[1,3-propanediylbis [(dimethyliminio)-3, 1-propanediyl-1 (4H)-pyridinyl-4- ylidene-1-propen-1-yl-3- ylidene]]bis[3-methyl]-, tetraiodide(POPOTM-3), EtBr(3,8-diamino-5-ethyl-6-phenyl-bromide), 3-methyl-2-[[1-[3-(trimethylammonio) propyl]-4(1H)-pyridinylidene]methyl]-, diiodide(PO-PRO™ -1), 3-methyl-2-[3-[1-[3-(trimethylammonio) propyl]-4(1H)-pyridinylidene]- 1-propenyl]-, diiodide(PO-PRO™ -3), and SYBR green(Molecular Probes). The acridine compound can be selected from the group consisting of 3,6-diaminoacridine, 3,6-diamino-10-methylacridinium chloride, acridine orange, and acridine yellow.

A preferred detection method of nucleic acid hybridization of the present invention comprises (a) preparing an oligo-plate by fixing capture probes on a working electrode surface, (b) hybridizing the capture probes with target probes, (c) reacting a nucleic acid binding material specific to single-stranded nucleic acid or a double-stranded nucleic acid, and (d) measuring an change of charge state on the working electrode surface with an electrochemical method under an electroactive electrolyte condition.

The detection method of nucleic acid hybridization further comprises a step of reacting the oligo-plate with a surfactant before step (c). The surfactant can be used as a liquid, dissolved in the same buffer used in the intercalation solution. The concentration of the surfactant is not limited, but it is preferably about 10 mM. The surfactant used can be a common surfactant, for example sodium dodecyl sulfate or triton-x.

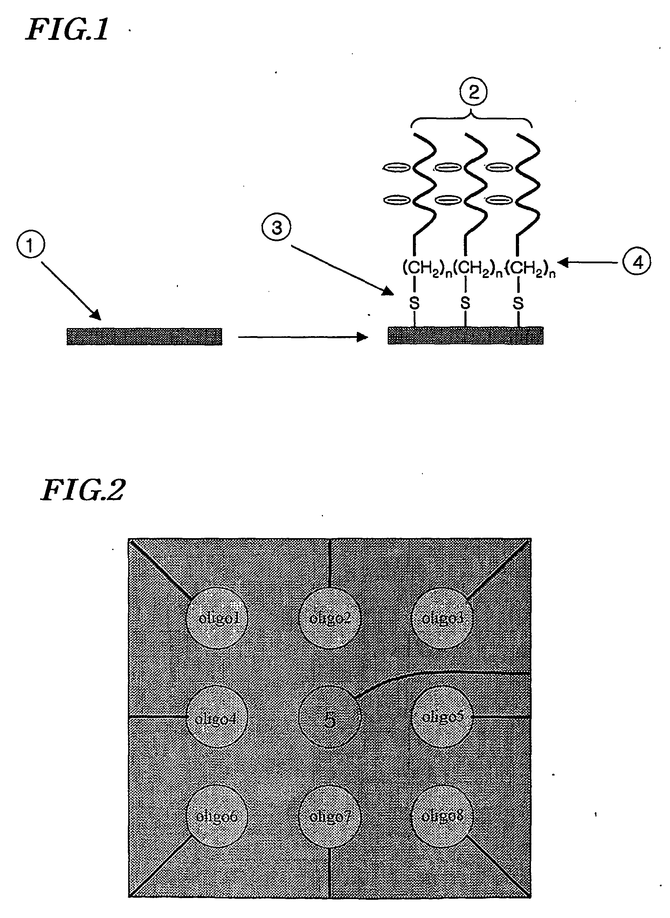

The oligo-plate is explained in more detail with reference to the example of Fig. 1. The oligo-plate can be prepared by fixing capture probes (②) to the working electrode surface (①) or by chemical bonding between the electrode surface and a thiol group (③) or amine group linked to the 5'or 3'-terminus of the capture probes. In addition, in order to prevent direct contact between the capture probe and the working electrode surface, a carbon chain linker (④) is further inserted there between. The carbon number of the carbon chain linker is not limited, but it is preferably 6 to 12.

The capture probe and target probe are DNA or RNA, and the working electrode is a conductive metal selected from the group consisting of gold, platinum, silver, carbon, copper, nickel, chromium, and palladium. The working electrode can be a thin film or a bar, and it can be prepared by plating a common matrix of a biochip with the metal.

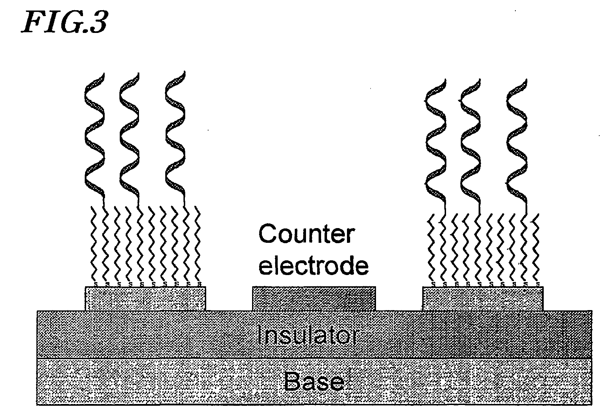

The working electrode of the present invention can have one capture probe per electrode fixed thereto. Therefore, for fixing several capture probes, a multi-array (Figs. 2 and 3) with multiple layers can be prepared. The multi-array may have auxiliary electrodes (⑤) at regular intervals. The auxiliary electrode is also known as a counter electrode, and it is a blank electrode without a capture probe fixed thereto, and gold or platinum can be used as the matrix. The auxiliary electrode prevents a voltage drop between the reference electrode and the working electrode during a chemical reaction, and it is conducive to delivery of a precise and constant voltage at the working electrode. The reference electrode provides a standard capable of controlling the magnitude of the voltage, and is usually made from silver/silver chloride (Ag/AgCl), produced by doping silver with AgCl. The reference electrode can be located at other places in the solution. The reference electrode also maintains a constant voltage irrespective of being around an electric reaction.

In the hybridizing of step (b) of the detection method of a nucleic acid hybridization, when the target probe is reacted with a capture probe that is fixed on the surface of the working electrode, a complementary capture probe for a target probe generates a nucleic acid hybrid (Fig. 4a), but a non-complementary capture probe does not bind with the target probe so it maintains a single-stranded nucleic acid.

The hybridization reaction can be performed according to the nature of the probe, its size, and so on, and more preferably, the hybridization reaction is conducted by a general protocol. In order to stabilize the nucleic acid hybrid between the capture probe and the target probe, a buffer containing sodium chloride can be used. After hybridization, the remaining non-specific binding target probe and the target probe are removed.

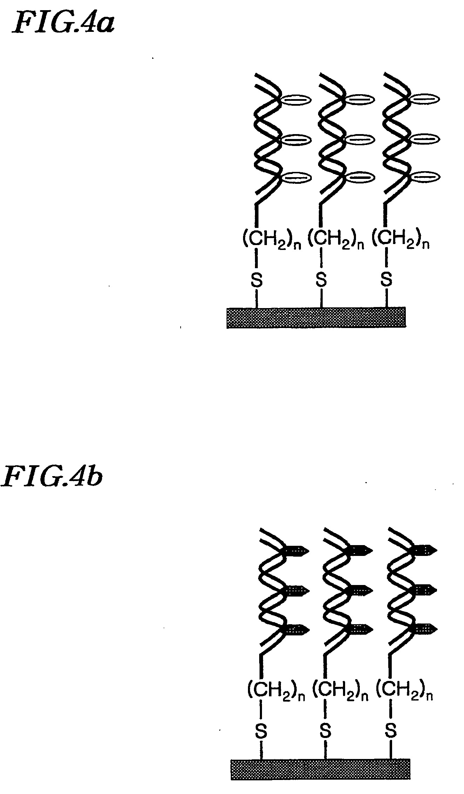

In the reaction of step (c), a single-stranded nucleic acid specific or a double-stranded nucleic acid specific nucleic acid binding material reacts with the capture probe or the nucleic hybrid on the surface of the working electrode. Therefore, the positively charged nucleic acid binding material is specifically bound with the nucleic hybrid or single-stranded nucleic acid to reduce negative charges (Fig. 4b).

Meanwhile, a surfactant, a heat, a voltage, or a sonic wave is applied so as to minimize non-specific binding after step (c). For example, a double-stranded nucleic acid specific intercalator is inserted to a staked base pair, but it also has a tendency to generate an ionic interaction with a single-stranded nucleic acid. This non-specific binding can be removed by applying with a surfactant, heat, sonic waves, or a voltage at the electrode surface. The temperature of the heat is preferably 65 to 95 °C, the treating with sonic waves can be performed by a general method or by sonication, and a voltage can be supplied to the electrode.

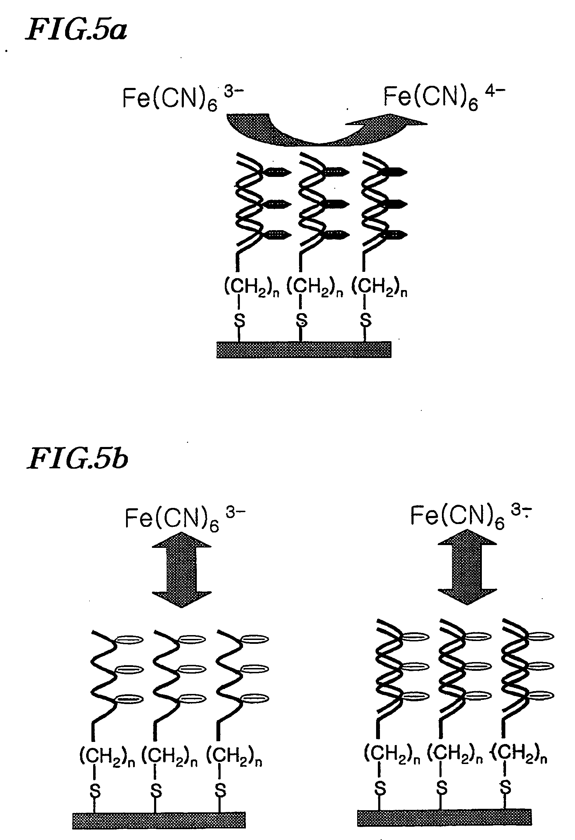

In step (d), the oligo-plate is immersed in an electrolytic condition to measure a change of charge state on the surface of the working electrode by an electrochemical method. At this time, if a negative charge of the nucleotides existing on the surface electrode is reduced or neutralized by nucleic acid binding material, a negatively charged electrolyte is allowed to go near the electrode surface, so the surface concentration of the negatively charged electrolyte increases and oxidation/reduction occurs. But if the negative charge of the nucleotides has not been reduced or neutralized, the negatively charged electrolyte is pushed out by electrostatic repulsion from the electrode surface, and oxidation/reduction does not occur (Fig. 5b).

The electrolyte is a negative compound, and it is preferably a solution containing [Fe(CN)₆]³⁻. An example of the electrolyte is a buffer including 50 mM NaCl, 10 mM phosphate (pH 7.0), and 3 mM K₃Fe(CN)₆.

Examples of the electrochemical method are cyclic voltammetry, amperometry, and chronocoulometry. Cyclic voltammetry is an electrochemical technique for studying variable potential at an electrode, involving application of a potential sweep followed by a sweep back through the potential region just covered, producing a triangular waveform. Amperometry uses techniques which involve measuring electric currents, and chronocoulometry is an electroanalysis performed by measuring the rate of change of current versus time at a working electrode.

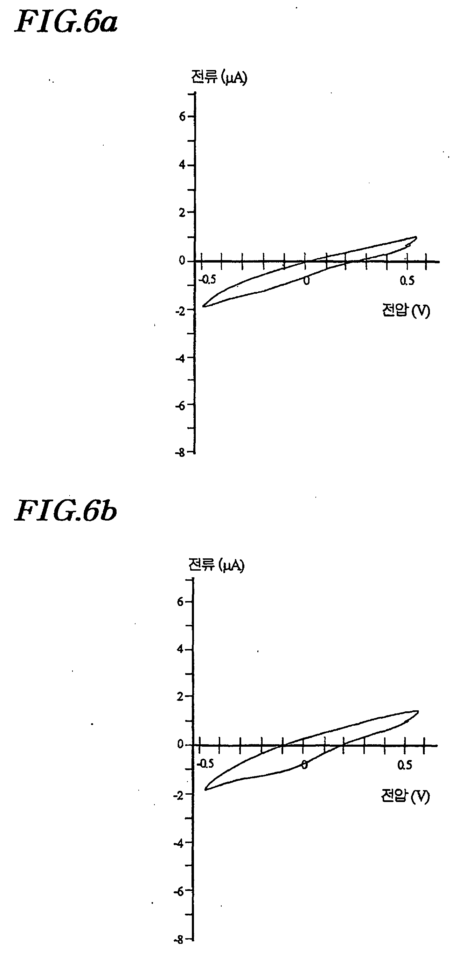

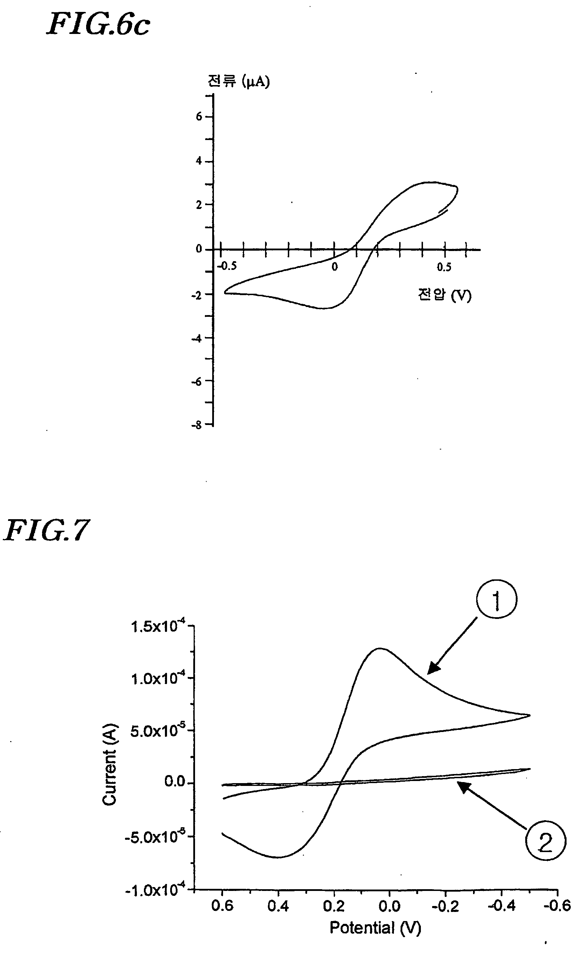

As examples, when a change of current at a working electrode is measured by cyclic voltammetry, a cyclic voltammogram at a working electrode before intercalation is produced as in Fig. 6a, a cyclic voltammogram at a working electrode fixed with non-intercalated DNA strand after intercalation is produced as in Fig. 6b, and a cyclic voltammogram at a working electrode fixed with intercalated DNA strand is produced as in Fig. 6c. That is, an intercalated capture probe has the negative charge on the electrode surface neutralized or reduced, so that the negatively charged electrolyte can easily approach the electrode surface, and occurrence of the reduction/oxidation is easy.

Therefore, when a double-stranded nucleic acid specific intercalator is reacted with a capture probe, if a cyclic voltammogram as in Fig. 6b is obtained, it means that the capture probe is non-complementary for the target probe. But if a cyclic voltammogram as in Fig. 6c is obtained, it means that the capture probe is complementary for the target probe.

As mentioned above, the detection method of nucleic acid hybridization of the present invention provides an electrochemical method for detecting nucleic acid hybridization, and this technique can be used for DNA chip or bio chips, with a low cost and a simple process.

The following examples are intended to further illustrate the present invention. However, these examples are shown only for better understanding of the present invention without limiting its scope.

### EXAMPLE 1. PREPARATION OF OLIGO-PLATE

A gold bar was used as a working electrode.

The gold bar was first polished with Nos. 1500 and 2000 sand paper, and then it was serially polished with alumina pastes of 5 µm, 1 µm, and 0.05 µm particle diameters, followed by sonication in deionized water for 5 min.

The electrode surface was boiled in a saturated potassium hydroxide solution for 1 hour, soaked in sulfuric acid for 24 hours, and thoroughly washed with deionized water. Instead of soaking in sulfuric acid, the electrode surface can be soaked in a piranha solution for 3-4 hours.

Oligonucleotides including a thiol group linked to 5' for capture probes were purchased from Sigma-Aldrich, with the sequence given in SEQ ID NO: 1.

10 ng/mL of the capture probe were dissolved in distilled water or 10 mM of a phosphate solution (pH 7.0) containing 50 mM of NaCl. The mixture was dripped on the gold electrode surface and incubated for more than 8 hours, followed by rinsing with distilled water. The gold electrode was then soaked in 10 mM of a phosphate solution (pH 7.0) containing 50 mM of NaCl, and after 24 hours it was washed with the same solution, thus preparing the oligo-plate.

### EXAMPLE 2. HYBRIDIZATION

Complementary oligonucleotides for a-capture probe were used as a target probe, and the target probe had the sequence given in SEQ ID NO: 2.

10 ug/ml of the target probe were added to a buffer (1X TE, 400mM NaCl, pH7.0) to prepare the target probe solution. The oligo-plate prepared in EXAMPLE 1 was immersed in the target probe solution for 15 hours, at 45 °C. After reducing the temperature, the oligo-plate was washed with 10 mM of a phosphate buffer (pH 7.0) containing 50 mM NaCl, and after the oligo-plate was soaked in 1X TBE (pH,7.0) containing 7 mM SDS or 10 mM phosphate buffer (pH 7.0) containing 50 mM NaCl and 7 mM SDS, it was washed with 1 x TBE (pH 7.0).

The oligo-plate was soaked in 1 X TBE (pH 7:0) containing 10⁻⁷ M of SYBR green for 10 mins, and it was washed with 10 mM phosphate (pH 7.0) containing 50 mM NaCl.

### EXAMPLE 3.

Non-complementary oligonucleotides for a capture probe were used as a target probe, and the target probe had the sequence given in SEQ ID NO: 3. The target probe of SEQ ID NO: 3 was reacted to the oligo-plate of EXAMPLE 1 by the same method as described in Example 2.

### EXAMPLE 4.

The cyclic voltammetry method was carried out on electrodes of EXAMPLE 2 and EXAMPLE 3 using an EG&G Potentiostat/Galvanostat Model 273A (Princeton Applied Research) at room temperature. An Ag/AgCl [3 M NaCl] reference electrode (BAS) and platinum wire auxiliary electrodes were used for the cyclic voltammetry measurement, and a [Fe(CN)₆]³⁻ solution containing 50 mM NaCl, 10 mM phosphate (pH 7.0), and 3 mM K₃Fe(CN)₆) was used as a buffer solution. The voltage scan range was 0.6V ~ -0.5V (versus an Ag/AgCl reference electrode), and the scan rate was 100 mV/sec. Oxygen was eliminated from the [Fe(CN)₆]³⁻solution by bubbling nitrogen therein for 5 minutes before the experiment.

Figs. 6a-c show cyclic voltammograms, wherein Fig. 6a is a cyclic voltammogram of a bare gold electrode, Fig. 6b is a cyclic voltammogram of the electrode of EXAMPLE 3, and Fig. 6c is a cyclic voltammogram of the electrode of EXAMPLE 2.

In Figs. 6a-c, it is shown that the capture probe hybridized with the target probe of EXAMPLE 2, but the capture probe could not hybridize with the non-complementary target probe of EXAMPLE 3 and the capture probe remained as a single strand DNA. These results are as predicted.

### EXAMPLE 5. HYBRIDIZATION DETECTION BY ACRIDINE COMPOUND.

An oligo-plate was prepared by the same method as described in EXAMPLE 1, and the hybridization reaction was performed. The oligo-plated was soaked in a buffer containing 0.5 ~ 5 mM SDS, 50 mM NaCl, and 10 mM phosphate (pH 7.0) for 10 mins. After washing with 50 mM NaCl and 10 mM phosphate (pH 7.0), the oligo-plate was soaked in 1 mg/mL 3,6-diaminoacridine, 50 mM NaCl, and 10 mM phosphate (pH 7.0) for 10 mins, and when it was clean, the cyclic voltammetry was measured.

Fig. 7 is a cyclic voltammogram of a working electrode after intercalation with an acridine compound. In the case of a working electrode fixed with a capture probe generating a DNA hybrid, a cyclic voltammogram as described in ① was measured, and with a working electrode fixed with only a single strand DNA, a cyclic voltammogram as described in ② was measured.

## Claims

1. An electrochemical method of detecting hybridization between a nucleic acid fixed on a electrode surface (capture probe) and a nucleic acid complementary thereto which is derived from a sample by electrically neutralizing a double stranded nucleic acid or a single stranded nucleic acid with a nucleic acid binding material,
wherein the detection method comprises:
(a) fixing the capture probe onto a electrode surface to obtain a nucleic acid layer;
(b) contacting the capture probe fixed on the electrode surface with the nucleic acid derived from sample to induce hybridization;
(c) simultaneously or sequentially reacting a hybridized or non-hybridized capture probe with a surfactant and a nucleic acid binding material, where the nucleic acid binding material has a positive charge, to change a charge amount or charge state of the hybridized or non-hybridized capture probe by binding thereto; and
(d) monitoring an electrical current or charge amount of the electrode surface in electroactive electrolyte solution to determine whether the capture probe is hybridized or not.

2. The method according to claim 1, wherein the nucleic acid is DNA or RNA.

3. The method according to claim 1, wherein the capture probe is fixed onto the electrode surface using a thiol group located at the terminus of the capture probe.

4. The method according to claim 1, wherein the capture probe is fixed onto the electrode surface using an amine group located at the terminus of the capture probe.

5. The method according to claim 1, wherein the electrode is made of a material selected from the group consisting of gold, platinum, silver, carbon, copper, nickel, chromium, and palladium.

6. The method according to claim 1, wherein the nucleic acid binding material is selected from the group consisting of an intercalator, an intercalating fluorescent dye, an acridine compound, chloroquine, quinine, novanatrone, doxorubicin, a single strand DNA binding protein, and Rec A protein.

7. The method according to claim 6, wherein the intercalating fluorescent dye is selected from the group consisting of
1,1'-[1,3-propanediylbis[(dimethyliminio)-3,1-propanediyl]]bis[4-[(3-methyl-2(3H)-benzoxa-zolylidene)methyl]]-,tetraiodide,
1,1'-[1,3-propanediylbis[(dimethyliminio)-3,1-propanediyl]]bis[4-[3-(3-methyl-2(3H)-benzoxazolylidene)-1-propenyl]]-,tetraiodide,
4-[(3-methyl-2(3H)-benzoxazolylidene)methyl]-1-[3-(trimethylammonio)propyl]-,diiodide,
4-[3-(3-methyl-2(3H)-benzoxazolylidene)-1-propenyl]-1-[3-(trimethylammonio)propyl]-,diiodide,
2,2'-[1,3-propanediylbis[(dimethyliminio)-3,1-propanediyl-1 (4H)-pyridinyl-4-ylidenemethylidyne]]bis[3-methyl]-,tetraiodide,
2,2'-[1,3-propanediylbis[(dimethyliminio)-3,1-propanediyl-1(4H)-pyridinyl-4-ylidene-1-propen-1-yl-3-ylidene]]bis[3-methyl]-,tetraiodide),
EtBr (3,8-diamino-5-ethyl-6-phenyl-bromide),
3-methyl-2-[[1-[3-(trimethylammonio)propyl]-4(1H)-pyridinylidene]methyl]-,diiodide,
3-methyl-2-[3-[1-[3-(trimethylammonio)propyl]-4(1H)-pyridinylidene]-1-propenyl]-,diiodide, and
SYBR green.

8. The method according to claim 6, wherein the acridine compound is selected from the group consisting of 3,6-diaminoacridine, 3,6-diamino-10-methylacridinium chloride, acridine orange, and acridine yellow.

9. The method according to claim 1, wherein the surfactant is sodium dodecyl sulfate or triton-X.

10. The method according to claim 1, wherein the detection method further comprises the step of applying heat, voltage, or sonic waves onto the nucleic acid layer after the step (c).

11. The method according to claim 1, wherein the electrochemical method is cyclic voltammetry, amperometry, or chronocoulometry.

12. The method according to claim 1, wherein the capture probe is fixed onto the electrode surface by chemical binding.

## Patentansprüche

1. Elektrochemisches Verfahren zum Nachweisen von Hybridisierung zwischen einer auf einer Elektrodenoberfläche fixierten Nukleinsäure (Capture-Sonde) und einer hierzu komplementären Nukleinsäure, die aus einer Probe stammt, indem man eine doppelsträngige Nukleinsäure oder eine einzelsträngige Nukleinsäure mit einem Nukleinsäure-bindenden Material elektrisch neutralisiert,
wobei das Nachweisverfahren umfasst:
(a) Fixieren der Capture-Sonde auf einer Elektrodenoberfläche, um eine Nukleinsäureschicht zu erhalten;
(b) In-Kontakt-Bringen der auf der Elektrodenoberfläche fixierten Capture-Sonde mit der aus der Probe stammenden Nukleinsäure, um eine Hybridisierung zu induzieren;
(c) Reagieren lassen einer hybridisierten oder nicht-hybridisierten Capture-Sonde, gleichzeitig oder nacheinander, mit einem Tensid und einem Nukleinsäure-bindenden Material, wobei das Nukleinsäure-bindende Material eine positive Ladung hat, um eine Ladungsmenge oder einen Ladungszustand der hybridisierten oder nicht-hybridisierten Capture-Sonde durch Binden daran zu verändern; und
(d) Monitoring eines elektrischen Stroms oder einer elektrischen Ladungsmenge von der Elektrodenoberfläche in elektroaktiver Elektrolytlösung, um zu bestimmen, ob die Capture-Sonde hybridisiert ist oder nicht.

2. Verfahren nach Anspruch 1, wobei die Nukleinsäure DNA oder RNA ist.

3. Verfahren nach Anspruch 1, wobei die Capture-Sonde mit einer Thiolgruppe am Terminus der Capture-Sonde auf der Elektrodenoberfläche fixiert wird.

4. Verfahren nach Anspruch 1, wobei die Capture-Sonde mit einer Aminogruppe am Terminus der Capture-Sonde auf der Elektrodenoberfläche fixiert wird.

5. Verfahren nach Anspruch 1, wobei die Elektrode aus einem Material hergestellt ist, das ausgewählt ist aus der Gruppe bestehend aus Gold, Platin, Silber, Kohle, Kupfer, Nickel, Chrom und Palladium.

6. Verfahren nach Anspruch 1, wobei das Nukleinsäure-bindende Material ausgewählt ist aus der Gruppe bestehend aus einem Interkalator, einem interkalierenden Fluoreszenzfarbstoff, einer Acridinverbindung, Chloroquin, Chinin, Novanatron, Doxorubicin, einem einzelsträngige DNA bindenden Protein und Rec A-Protein.

7. Verfahren nach Anspruch 6, wobei der interkalierende Fluoreszenzfarbstoff ausgewählt ist aus der Gruppe bestehend aus
1,1'-[1,3-Propandiylbis[(dimethyliminio)-3,1-propandiyl]]bis[4-[(3-methyl-2(3H)-benzoxazolyliden)methyl]]-,tetradiodid,
1,1'-[1,3-Propandiylbis[(dimethyliminio)-3,1-propandiyl]]bis[4-[3-(3-methyl-2(3H)-benzoxazolyliden)-1-propenyl]]-,tetradiodid,
4-[(3-Methyl-2(3H)-benzoxazolyliden)methyl]-1-[3-(trimethylammonio)propyl]-,diiodid,
4-[3-(3-Methyl-2(3H)-benzoxazolyliden)-1-propenyl]-1-[3-(trimethylammonio)propyl]-,diiodid,
2,2'-[1,3-Propandiylbis[(dimethyliminio)-3,1-propandiyl-1(4H)-pyridinyl-4-ylidenmethylidyn]]bis[3-methyl]-,tetradiodid,
2,2'-[1,3-Propandiylbis[(dimethyliminio)-3,1-propandiyl-1(4H)-pyridinyl-4-yliden-1-propen-1-yl-3-yliden]]bis[3-methyl]-,tetradiodid,
EtBr (3,8-Diamino-5-ethyl-6-phenylbromid),
3-Methyl-2-[[1-[3-(trimethylammonio)propyl]-4(1H)-pyridinyliden]methyl]-,diiodid,
3-Methyl-2-[3-[1-[3-(trimethylammonio)propyl]-4(1H)-pyridinyliden]-1-propenyl]-,diiodid, und
SYBR-Green.

8. Verfahren nach Anspruch 6, wobei die Acridinverbindung ausgewählt ist aus der Gruppe bestehend aus 3,6-Diaminoacridin, 3,6-Diamino-10-methylacridiniumchlorid, Acridinorange und Acridingelb.

9. Verfahren nach Anspruch 1, wobei das Tensid Natriumdodecylsulfat oder Triton-X ist.

10. Verfahren nach Anspruch 1, wobei das Nachweisverfahren nach Schritt (c) ferner den Schritt des Zuführens von Wärme, Spannung oder Schallwellen auf die Nukleinsäureschicht umfasst.

11. Verfahren nach Anspruch 1, wobei das elektrochemische Verfahren cyclische Voltammetrie, Amperometrie oder Chronocoulometrie ist.

12. Verfahren nach Anspruch 1, wobei die Capture-Sonde durch chemische Bindung auf der Elektrodenoberfläche fixiert wird.

## Revendications

1. Procédé électrochimique de détection d'une hybridation entre un acide nucléique fixé sur une surface d'électrode (sonde de capture) et un acide nucléique qui lui est complémentaire qui est dérivé d'un échantillon par neutralisation électrique d'un acide nucléique bicaténaire ou d'un acide nucléique monocaténaire avec un matériau de liaison d'acide nucléique ;
dans lequel le procédé de détection comprend les étapes consistant à :
(a) fixer la sonde de capture sur une surface d'électrode pour obtenir une couche d'acide nucléique ;
(b) mettre en contact la sonde de capture fixée sur la surface d'électrode avec l'acide nucléique dérivé de l'échantillon pour provoquer l'hybridation ;
(c) mettre en réaction simultanément ou séquentiellement une sonde de capture hybridée ou non hybridée avec un surfactant et un matériau de liaison d'acide nucléique, où le matériau de liaison d'acide nucléique présentant une charge positive, pour modifier une quantité de charge ou un état de charge de la sonde de capture hybridée ou non hybridée par liaison à celui-ci ; et
(d) surveiller un courant électrique ou une quantité de charge de la surface d'électrode dans une solution d'électrolytes électro-active pour déterminer si la sonde de capture est hybridée ou non.

2. Procédé selon la revendication 1, dans lequel l'acide nucléique est de l'ADN ou de l'ARN.

3. Procédé selon la revendication 1, dans lequel la sonde de capture est fixée sur la surface d'électrode en utilisant un groupe thiol situé à l'extrémité de la sonde de capture.

4. Procédé selon la revendication 1, dans lequel la sonde de capture est fixée sur la surface d'électrode en utilisant un groupe aminé situé à l'extrémité de la sonde de capture.

5. Procédé selon la revendication 1, dans lequel l'électrode est fabriquée en un matériau choisi dans le groupe comprenant l'or, le platine, l'argent, le carbone, le cuivre, le nickel, le chrome et le palladium.

6. Procédé selon la revendication 1, dans lequel le matériau de liaison à l'acide nucléique est choisi dans le groupe consistant en un intercalateur, un colorant fluorescent intercalant, un composé acridine, la chloroquine, la quinine, la novanatrone, la doxorubicine, une protéine de liaison d'ADN monocaténaire, et la protéine Rec A.

7. Procédé selon la revendication 6, dans lequel le colorant fluorescent intercalant est choisi dans le groupe comprenant :
la 1,1'-[1,3-propanediylbis[(diméthyliminio)-3,1-propanediyl]]bis[4-[(3-méthyl-2(3H)-benzoxazolylidène)méthyl]]-,tétraiodure,
la 1,1'-[1,3-propanediylbis[(diméthyliminio)-3,1-propanediyl]]bis[4-[3-(3-méthyl-2(3H)-benzoxazolylidène)-1-propényl]]-,tétraiodure,
la 4-[(3-méthyl-2(3H)-benzoxazolylidène)méthyl]-1-[3(triméthylammonio)propyl]-,diiodure,
la 4-[3-(3-méthyl-2(3H)-benzoxazolylidène)-1-propényl]-1-[3(triméthylammonio)propyl]-,diiodure,
la 2,2'-[1,3-propanediylbis[(diméthyliminio)-3,1-propanediyl-1(4H)pyridinyl-4-ylidèneméthylidyne]]bis[3-méthyl]-,tétraiodure,
la 2,2'-[1,3-propanediylbis[(diméthyliminio)-3,1-propanediyl-1(4H)-pyridinyl-4-ylidène-1-propén-1-yl-3-ylidène]]bis[3-méthyl]-, tetraiodure),
l'EtBr (3,8-diamino-5-éthyl-6-phényl-bromure),
la 3-méthyl-2-[[1-[3-(triméthylammonio)propyl]-4(1H)-pyridinylidène]méthyl]-,diiodure,
la 3-méthyl-2-[3-[1-[3-(triméthylammonio)propyl]-4(1H)-pyridinylidène]-1-propényl]-,diiodure, et
le vert SYBR

8. Procédé selon la revendication 6, dans lequel le composé acridine est choisi dans le groupe comprenant la 3,6-diaminoacridine, le chlorure de 3,6-diamino-10-méthylacridinium, l'orange acridine et le jaune acridine.

9. Procédé selon la revendication 1, dans lequel le surfactant est le dodécylsulfate de sodium ou le triton-X.

10. Procédé selon la revendication 1, dans lequel le procédé de détection comprend en outre l'étape consistant à appliquer de la chaleur, une tension ou des ondes soniques sur la couche d'acide nucléique après l'étape (c).

11. Procédé selon la revendication 1, dans lequel le procédé électrochimique est la voltammétrie cyclique-, l'ampérométrie ou la chronocoulométrie.

12. Procédé selon la revendication 1, dans lequel la sonde de capture est fixée à la surface de l'électrode par liaison chimique.


### Sequence Listing

<110> POHANG IRON STEEL CO., LTD.
   Pohang University of Science and Technology
<120> DETECTION METHOD OF NUCLEIC ACID HYBRIDIZATION
<130> DPP021550
<150> KR 10-2001-44971
   <151> 2001-07-25
<150> KR 10-2002-43389
   <151> 2002-07-23
<160> 3
<170> KopatentIn 1.71
<210> 1
   <211> 20
   <212> DNA
   <213> Artificial Sequence
<220>
   <223> sequence of capture probe
<400> 1
   gttttcccag tcacgacggg 20
<210> 2
   <211> 20
   <212> DNA
   <213> Artificial Sequence
<220>
   <223> sequence of target probe
<400> 2
   cccgtcgtga ctgggaaaac 20
<210> 3
   <211> 20
   <212> DNA
   <213> Artificial Sequence
<220>
   <223> sequence of target probe
<400> 3
   ctcgccatgt ctgtgaccac 20